(19) Europäisches Patentamt
European Patent Office
Office européen des brevets

(11) **EP 4 241 579 A1**

(12) **EUROPEAN PATENT APPLICATION**
published in accordance with Art. 153(4) EPC

(43) Date of publication:
**13.09.2023 Bulletin 2023/37**

(21) Application number: **21877982.5**

(22) Date of filing: **06.10.2021**

(51) International Patent Classification (IPC):
***A23L 33/10*** *(2016.01)* ***A23L 33/105*** *(2016.01)*
***A23L 5/20*** *(2016.01)* ***A23L 29/00*** *(2016.01)*
***A61K 31/265*** *(2006.01)* ***A61K 31/404*** *(2006.01)*
***A61K 31/575*** *(2006.01)*

(52) Cooperative Patent Classification (CPC):
**A23L 5/20; A23L 29/00; A23L 33/10; A23L 33/105; A61K 31/265; A61K 31/404; A61K 31/575**

(86) International application number:
**PCT/KR2021/013668**

(87) International publication number:
**WO 2022/075731 (14.04.2022 Gazette 2022/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.10.2020 KR 20200129117**
**07.10.2020 KR 20200129118**

(71) Applicant: **Astrogenesis Co., Ltd.**
**Seoul 04110 (KR)**

(72) Inventors:
- **LEE, Kang Hyun**
  **Seoul 04129 (KR)**
- **KUK, Min**
  **Seoul 04129 (KR)**

(74) Representative: **Bates, Philip Ian**
**Reddie & Grose LLP**
**The White Chapel Building**
**10 Whitechapel High Street**
**London E1 8QS (GB)**

(54) **COMPOSITION FOR PROMOTING HAIR GROWTH AND/OR INHIBITING HAIR LOSS**

(57) The present application relates to a composition including a herb extract and/or an effective compound as an active ingredient for promoting hair growth and/or preventing, alleviating, or treating hair loss.

【FIG. 1a】

2500
2000
1500
1000
500
0
Testosterone (pg/ml)
Group 1
Group 2
Group 3
Group 4
Group 5



Processed by Luminess, 75001 PARIS (FR)

## Description

[TECHNICAL FIELD]

Cross reference to related application(s)

The present application claims the benefit of Korean patent application No. 10-2020-0129117 filed on October 7, 2020, and Korean patent application No. 10-2020-0129118 filed on October 7, 2020, and the entire contents disclosed in the documents of the corresponding Korean patent applications are incorporated as a part of the present specification.

The present application relates to a composition for promoting hair growth and/or inhibiting hair loss comprising an herbal extract and/or an effective compound as an active ingredient.

[BACKGROUND ART]

Human hair originates from hair follicles that exist within the scalp. Since hair follicles are originally formed in the fetus and are no longer produced after birth, the number of hair follicles remains constant from adolescence to adulthood, and then gradually decreases from old age. Human hair repeats growth and extinction through a unique cycle of anagen stage, catagen stage and telogen stage. This hair cycle is repeated over 3 to 6 years and it is known that in addition to genetic factors, it is affected by various factors such as age, season, disease, stress and the like. In general, alopecia refers to an abnormal increase in the number of hairs falling out due to an increase in hair in the catagen stage or telogen stage while the number of hairs in the anagen stage decreases during the aforementioned hair cycle.

In general, hair loss is due to heredity, aging, disease, taking medicine, radiation therapy, and the like, but from a biochemical or physiological point of view, the causes of hair loss are excessive male hormone secretion, excessive sebum secretion, scalp blood circulation disorder, nutrient deficiency, nutritional imbalance, stress and the like. However, the exact cause and mechanism of hair loss have not been identified yet, and there is also a shortage of clear methods or drugs to treat alopecia.

[DISCLOSURE]

[TECHNICAL PROBLEM]

One example of the present application provides a composition for promoting hair growth, comprising one or more kinds selected from the group consisting of sulforaphane, indole-3-carbionol, beta-sitosterol (β-sitosterol), and salts thereof as an active ingredient.

Another example provides a composition for promoting hair growth, comprising a broccoli (Brassica oleracea var. italica) extract, an avocado (Persea americana) extract or a mixture thereof as an active ingredient.

The composition for promoting hair growth may be a pharmaceutical composition, a cosmetic composition and/or a food composition (for example, health functional food).

Other example provides a pharmaceutical composition for preventing, alleviating or treating hair loss, comprising one or more kinds selected from the group consisting of sulforaphane, indole-3-carbionol, beta-sitosterol (β-sitosterol), and salts thereof as an active ingredient.

Other example provides a pharmaceutical composition for preventing, alleviating or treating hair loss, comprising a broccoli extract, an avocado extract or a mixture thereof as an active ingredient.

Other example provides a cosmetic composition for preventing, alleviating or treating hair loss, comprising one or more kinds selected from the group consisting of sulforaphane, indole-3-carbionol, beta-sitosterol, and salts thereof as an active ingredient.

Other example provides a cosmetic composition for preventing, alleviating or treating hair loss, comprising a broccoli extract, an avocado extract or a mixture thereof as an active ingredient.

Other example provides a food composition for preventing, alleviating or treating hair loss, comprising one or more kinds selected from the group consisting of sulforaphane, indole-3-carbionol, beta-sitosterol, and salts thereof as an active ingredient. The food composition may be a health functional food.

Other example provides a food composition for preventing, alleviating or treating hair loss, comprising a broccoli extract, an avocado extract or a mixture thereof as an active ingredient. The food composition may be a health functional food. Other example provides a method for promoting hair growth and/or preventing, alleviating and/or treating hair loss comprising administering an effective dose of one or more kinds selected from the group consisting of sulforaphane, indole-3-carbionol, beta-sitosterol, and salts thereof to a subject in need of promoting hair growth and/or preventing, alleviating and/or treating hair loss.

Other example provides a method for promoting hair growth and/or preventing, alleviating and/or treating hair

loss comprising administering an effective dose of a broccoli extract, an avocado extract or a mixture thereof to a subject in need of promoting hair growth and/or preventing, alleviating and/or treating hair loss.

Other example provides a use for using in promoting hair growth and/or preventing, alleviating or treating hair loss, or preparation of a composition for promoting hair growth and/or preventing, alleviating or treating hair loss, of one or more kinds selected from the group consisting of sulforaphane, indole-3-carbionol, beta-sitosterol, and salts thereof.

Other example provides a use for using in promoting hair growth and/or preventing, alleviating and/or treating hair loss, or preparation of a composition for promoting hair growth and/or preventing, alleviating and/or treating hair loss, of a broccoli extract, an avocado extract or a mixture thereof.

[TECHNICAL SOLUTION]

Herein, an herbal extract and a useful compound having an effect of promoting hair growth and/or inhibiting hair loss are confirmed, thereby providing a use in promoting hair growth and/or inhibiting hair loss thereof.

More specifically, one example provides a composition for promoting hair growth, comprising one or more kinds selected from the group consisting of sulforaphane, indole-3-carbionol, beta-sitosterol (β-sitosterol), and salts thereof as an active ingredient.

Another example provides a composition for promoting hair growth, comprising a broccoli (*Brassica oleracea* var. italica) extract, an avocado *(Persea americana)* extract or a mixture thereof as an active ingredient.

The composition for promoting hair growth may be a pharmaceutical composition, a cosmetic composition and/or a food composition (for example, health functional food).

Other example provides a pharmaceutical composition for preventing, alleviating or treating hair loss, comprising one or more kinds selected from the group consisting of sulforaphane, indole-3-carbionol, beta-sitosterol and salts thereof as an active ingredient.

Other example provides a pharmaceutical composition for preventing, alleviating or treating hair loss, comprising a broccoli extract, an avocado extract or a mixture thereof as an active ingredient.

Other example provides a cosmetic composition for preventing, alleviating or treating hair loss, comprising one or more kinds selected from the group consisting of sulforaphane, indole-3-carbionol, beta-sitosterol, and salts thereof as an active ingredient.

Other example provides a cosmetic composition for preventing, alleviating or treating hair loss, comprising a broccoli extract, an avocado extract or a mixture thereof as an active ingredient.

Other example provides a food composition for preventing, alleviating or treating hair loss, comprising one or more kinds selected from the group consisting of sulforaphane, indole-3-carbionol, beta-sitosterol, and salts thereof as an active ingredient. The food composition may be a health functional food.

Other example provides a food composition for preventing, alleviating or treating hair loss, comprising a broccoli extract, an avocado extract or a mixture thereof as an active ingredient. The food composition may be a health functional food.

Other example provides a method for promoting hair growth and/or preventing, alleviating and/or treating hair loss comprising administering an effective dose of one or more kinds selected from the group consisting of sulforaphane, indole-3-carbionol, beta-sitosterol, and salts thereof to a subject in need of promoting hair growth and/or preventing, alleviating and/or treating hair loss.

Other example provides a method for promoting hair growth and/or preventing, alleviating and/or treating hair loss comprising administering an effective dose of a broccoli extract, an avocado extract or a mixture thereof to a subject in need of promoting hair growth and/or preventing, alleviating and/or treating hair loss.

Other example provides a use for using in promoting hair growth and/or preventing, alleviating or treating hair loss, or preparation of a composition for promoting hair growth and/or preventing, alleviating or treating hair loss, of one or more kinds selected from the group consisting of sulforaphane, indole-3-carbionol, beta-sitosterol, and salts thereof.

Other example provides a use for using in promoting hair growth and/or preventing, alleviating and/or treating hair loss, or preparation of a composition for promoting hair growth and/or preventing, alleviating and/or treating hair loss, of a broccoli extract, an avocado extract or a mixture thereof.

All numerical values or ranges of numerical values related to the composition and method provided herein may be selected to contribute to or exhibit more advantageous effects on the purpose of the composition and method, for example, promoting hair growth and/or inhibiting hair loss (prevention, improvement and/or treatment of hair loss).

Hereinafter, the present invention will be described in more detail.

Active compounds

The sulforaphane (SFN) is a compound having a chemical formula of $C_6H_{11}NOS_2$ (CAS No. 4478-93-7), and may have the structure of Chemical formula 1 below.

[Chemical formula 1]

The indole-3-carbinol (I3C) is a compound having a chemical formula of $C_9H_9NO$ (CAS No. 700-06-1), and may have the structure of Chemical formula 2 below.

[Chemical formula 2]

The β-sitosterol is a compound having a chemical formula of $C_{29}H_{50}O$ (CAS No. 83-46-5), and may have the structure of Chemical formula 3 below.

[Chemical formula 3]

The compounds of Chemical formula 1 to Chemical formula 3 or salts thereof may be obtained by extracting and/or isolating from a natural product and/or a strain, or prepared by a conventional organic synthesis method, but not limited thereto.

In the present application, "salt of a compound" may mean a physiologically acceptable salt among salts, which are substances in which cations and anions are combined by electrostatic attraction, and for example, it may mean a pharmaceutically acceptable salt, a cosmetically acceptable salt and/or a salt that is acceptable for food. For example, the salt may be one or more kinds selected from the group consisting of metal salts, salts with organic bases, salts with inorganic acids, salts with organic acids, salts with basic or acidic amino acids, and the like. In one example, the metal salts may be one or more kinds selected from the group consisting of alkali metal salts (sodium salt, potassium salt, etc.), alkali earth metal salts (potassium salt, magnesium salt, barium salt, etc.), aluminum salts, and the like; and the salts with organic bases may be one or more kinds selected from the group consisting of salts with triethylamine, pyridine, picoline, 2,6-lutidine, ethanolamine, diethanolamine, triethanolamine, cyclohexylamine, dicyclohexylamine, N,N-dibenzylethylenediamine, and the like; and the salts with inorganic acids may be one or more kinds selected from the group consisting of salts with hydrochloric acid, hydrobromic acid, nitric acid, sulfuric acid, phosphoric acid, and the like; and the salts with organic acids may be one or more kinds selected from the group consisting of formic acid, acetic acid, trifluoroacetic acid, phthalic acid, fumaric acid, oxalic acid, tartaric acid, maleic acid, citric acid, succinic acid, methanesulfonic acid, benzenesulfonic acid, p-toluenesulfonic acid, and the like; and the salts with basic amino acids may be one or more kinds selected from the group consisting of salts with arginine, lysine, ornithine, and the like; and the salts with acidic amino acids may be one or more kinds selected from the group consisting of salts with aspartic acid, glutamic acid, and the like.

In one example, the active ingredient may be sulforaphane.

In another example, the active ingredient may be indole-3-carbinol.

In other example, the active ingredient may be β-sitosterol.

In other example, the active ingredient may be a combination of sulforaphane and indole-3-carbinol. In this case, the ratio of sulforaphane and indole-3-carbinol contained in the composition or administered in the method provided herein may be 10: 0.1 to 10, 10: 0.1 to 7.5, 10: 0.1 to 5, 10: 0.1 to 2.5, 10: 0.1 to 2, 10: 0.1 to 1.5, 10: 0.1 to 1.25, 10: 0.25 to 10, 10: 0.25 to 7.5, 10: 0.25 to 5, 10: 0.25 to 2.5, 10: 0.25 to 2, 10: 0.25 to 1.5, 10: 0.25 to 1.25, 10: 0.5 to 10, 10: 0.5 to 7.5, 10: 0.5 to 5, 10: 0.5 to 2.5, 10: 0.5 to 2, 10: 0.5 to 1.5, 10: 0.5 to 1.25, 10: 0.75 to 10, 10: 0.75 to 7.5, 10: 0.75 to 5, 10: 0.75 to 2.5, 10: 0.75 to 2, 10: 0.75 to 1.5, 10: 0.75 to 1.25, 10: 1 to 10, 10: 1 to 7.5, 10: 1 to 5, 10: 1 to 2.5, 10: 1 to 2, 10: 1 to 1.5, 10: 1 to 1.25, 10: 1.25 to 10, 10: 1.25 to 7.5, 10: 1.25 to 5, 10: 1.25 to 2.5, 10: 1.25 to 2, 10: 1.25 to 1.5, 10: 2.5 to 10, 10: 2.5 to 7.5, 10: 2.5 to 5, about 10: 1.25, or about 10: 2.5 (so far, sulforaphane weight: indole-3-carbinol weight), based on the weight.

In other example, the active ingredient may be a combination of sulforaphane and β-sitosterol. In this case, the ratio of sulforaphane and β-sitosterol contained in the composition or administered in the method provided herein may be 10: 0.1 to 10, 10: 0.1 to 7.5, 10: 0.1 to 5, 10: 0.1 to 2.5, 10: 0.1 to 2, 10: 0.1 to 1.5, 10: 0.1 to 1.25, 10: 0.25 to 10, 10: 0.25 to 7.5, 10: 0.25 to 5, 10: 0.25 to 2.5, 10: 0.25 to 2, 10: 0.25 to 1.5, 10: 0.25 to 1.25, 10: 0.5 to 10, 10: 0.5 to 7.5, 10: 0.5 to 5, 10: 0.5 to 2.5, 10: 0.5 to 2, 10: 0.5 to 1.5, 10: 0.5 to 1.25, 10: 0.75 to 10, 10: 0.75 to 7.5, 10: 0.75 to 5, 10: 0.75 to 2.5, 10: 0.75 to 2, 10: 0.75 to 1.5, 10: 0.75 to 1.25, 10: 1 to 10, 10: 1 to 7.5, 10: 1 to 5, 10: 1 to 2.5, 10: 1 to 2, 10: 1 to 1.5, 10: 1 to 1.25, 10: 1.25 to 10, 10: 1.25 to 7.5, 10: 1.25 to 5, 10: 1.25 to 2.5, 10: 1.25 to 2, 10: 1.25 to 1.5, 10: 2.5 to 10, 10: 2.5 to 7.5, 10: 2.5 to 5, about 10: 1.25, or about 10: 2.5 (so far, sulforaphane weight: β-sitosterol weight), based on the weight.

In other example, the active ingredient may be a combination of indole-3-carbinol and β-sitosterol. In this case, the ratio of indole-3-carbinol and β-sitosterol contained in the composition or administered in the method provided herein may be 1: 0.1 to 10, 1: 0.1 to 7.5, 1: 0.1 to 5, 1: 0.1 to 2.5, 1: 0.1 to 1, 1: 0.25 to 10, 1: 0.25 to 7.5, 1: 0.25 to 5, 1: 0.25 to 2.5, 1: 0.25 to 1, 1: 0.5 to 10, 1: 0.5 to 7.5, 1: 0.5 to 5, 1: 0.5 to 2.5, 1: 0.5 to 1, 1: 0.75 to 10, 1: 0.75 to 7.5, 1: 0.75 to 5, 1: 0.75 to 2.5, 1: 0.75 to 1, 1: 1 to 10, 1: 1 to 7.5, 1: 1 to 5, 1: 1 to 2.5, or about 1:1 (so far, indole-3-carbinol weight: β-sitosterol weight), based on the weight.

In other example, the active ingredient may be a combination of sulforaphane, indole-3-carbinol and β-sitosterol. In this case, the ratio of sulforaphane, indole-3-carbinol and β-sitosterol contained in the composition or administered in the method provided herein may be 10: 0.1 to 10: 0.1 to 10, 10: 0.1 to 7.5: 0.1 to 7.5, 10: 0.1 to 5: 0.1 to 5, 10: 0.1 to 2.5: 0.1 to 2.5, 10: 0.1 to 2: 0.1 to 2, 10: 0.1 to 1.5: 0.1 to 1.5, 10: 0.1 to 1.25: 0.1 to 1.25, 10: 0.25 to 10: 0.25 to 10, 10: 0.25 to 7.5: 0.25 to 7.5, 10: 0.25 to 5: 0.25 to 5, 10: 0.25 to 2.5: 0.25 to 2.5, 10: 0.25 to 2: 0.25 to 2, 10: 0.25 to 1.5: 0.25 to 1.5, 10: 0.25 to 1.25: 0.25 to 1.25, 10: 0.5 to 10: 0.5 to 10, 10: 0.5 to 7.5: 0.5 to 7.5, 10: 0.5 to 5: 0.5 to 5, 10: 0.5 to 2.5: 0.5 to 2.5, 10: 0.5 to 2: 0.5 to 2, 10: 0.5 to 1.5: 0.5 to 1.5, 10: 0.5 to 1.25: 0.5 to 1.25, 10: 0.75 to 10: 0.75 to 10, 10: 0.75 to 7.5: 0.75 to 7.5, 10: 0.75 to 5: 0.75 to 5, 10: 0.75 to 2.5: 0.75 to 2.5, 10: 0.75 to 2: 0.75 to 2, 10: 0.75 to 1.5: 0.75 to 1.5, 10: 0.75 to 1.25: 0.75 to 1.25, 10: 1 to 10: 1 to 10, 10: 1 to 7.5: 1 to 7.5, 10: 1 to 5: 1 to 5, 10: 1 to 2.5: 1 to 2.5, 10: 1 to 2: 1 to 2, 10: 1 to 1.5: 1 to 1.5, 10: 1 to 1.25: 1 to 1.25, 10: 1.25 to 10: 1.25 to 10, 10: 1.25 to 7.5: 1.25 to 7.5, 10: 1.25 to 5: 1.25 to 5, 10: 1.25 to 2.5: 1.25 to 2.5, 10: 1.25 to 2, 10: 1.25 to 1.5: 1.25 to 1.5, or 10: 1.25: 1.25 (so far, sulforaphane weight: indole-3-carbinol weight: β-sitosterol weight), based on the weight.

In one example, sulforaphane, indole-3-carbinol, β-sitosterol and/or salts thereof described as an active ingredient in the composition and/or method provided herein may be in a form comprised in a broccoli extract, an avocado extract or a mixture thereof or a separated (or purified) form therefrom.

Extract

Broccoli *(Brassica oleracea* var. italica) is an annual dicotyledonous plant belonging to the Mustard family of Papaver order, and is used in various ways for food. The broccoli extract used herein may be obtained by using one or more parts selected from the group consisting of broccoli seeds, buds, flowers, roots, whole plants and the like. The sprout may refer to the first stem and/or leaf generated from the seed. The broccoli used for extraction herein may be in the form of a raw material, a dried product, or a crushed product of the herbal medicine or dried product, but not limited thereto.

Avocado *(Persea americana)* is an annual dicotyledonous plant belonging to the camphor family of camphor order, and is a fruit native to Mexico and Central America. The avocado extract used herein may be obtained by using one or more parts selected from the group consisting of avocado seeds, fruits (flesh and/or peel), buds, flowers, stems, roots, whole plants, and the like. The sprout may refer to the first stem and/or leaf generated from the seed. The avocado used for extraction herein may be in the form of a raw material, a dried product, or a crushed product of the herbal medicine or dried product, but not limited thereto.

The broccoli extract may be obtained by extracting broccoli with one or more kinds of extraction solvents selected from the group consisting of water and straight-chain or branched alcohol having 1 to 4 carbon atoms. In one

example, the broccoli extract may be obtained by extracting broccoli with water, or 5 to 100%(v/v), 5 to 98%(v/v), 5 to 95%(v/v), 5 to 92%(v/v), 5 to 90%(v/v), 5 to 85%(v/v), 5 to 80%(v/v), 5 to 75%(v/v), 5 to 70%(v/v), 5 to 65%(v/v), 5 to 60%(v/v), 5 to 55%(v/v), 5 to 50%(v/v), 5 to 45%(v/v), 5 to 40%(v/v), 5 to 35%(v/v), 5 to 30%(v/v), 5 to 25%(v/v), 5 to 20%(v/v), 5 to 15%(v/v), 5 to 10%(v/v), 10 to 100%(v/v), 10 to 98%(v/v), 10 to 95%(v/v), 10 to 92%(v/v), 10 to 90%(v/v), 10 to 85%(v/v), 10 to 80%(v/v), 10 to 75%(v/v), 10 to 70%(v/v), 10 to 65%(v/v), 10 to 60%(v/v), 10 to 55%(v/v), 10 to 50%(v/v), 10 to 45%(v/v), 10 to 40%(v/v), 10 to 35%(v/v), 10 to 30%(v/v), 10 to 25%(v/v), 10 to 20%(v/v), 10 to 15%(v/v), 15 to 100%(v/v), 15 to 98%(v/v), 15 to 95%(v/v), 15 to 92%(v/v), 15 to 90%(v/v), 15 to 85%(v/v), 15 to 80%(v/v), 15 to 75%(v/v), 15 to 70%(v/v), 15 to 65%(v/v), 15 to 60%(v/v), 15 to 55%(v/v), 15 to 50%(v/v), 15 to 45%(v/v), 15 to 40%(v/v), 15 to 35%(v/v), 15 to 30%(v/v), 15 to 25%(v/v), 15 to 20%(v/v), 20 to 100%(v/v), 20 to 98%(v/v), 20 to 95%(v/v), 20 to 92%(v/v), 20 to 90%(v/v), 20 to 85%(v/v), 20 to 80%(v/v), 20 to 75%(v/v), 20 to 70%(v/v), 20 to 65%(v/v), 20 to 60%(v/v), 20 to 55%(v/v), 20 to 50%(v/v), 20 to 45%(v/v), 20 to 40%(v/v), 20 to 35%(v/v), 20 to 30%(v/v), 20 to 25%(v/v), 25 to 100%(v/v), 25 to 98%(v/v), 25 to 95%(v/v), 25 to 92%(v/v), 25 to 90%(v/v), 25 to 85%(v/v), 25 to 80%(v/v), 25 to 75%(v/v), 25 to 70%(v/v), 25 to 65%(v/v), 25 to 60%(v/v), 25 to 55%(v/v), 25 to 50%(v/v), 25 to 45%(v/v), 25 to 40%(v/v), 25 to 35%(v/v), 25 to 30%(v/v), 30 to 100%(v/v), 30 to 98%(v/v), 30 to 95%(v/v), 30 to 28%(v/v), 30 to 90%(v/v), 30 to 85%(v/v), 30 to 80%(v/v), 30 to 75%(v/v), 30 to 70%(v/v), 30 to 65%(v/v), 30 to 60%(v/v), 30 to 55%(v/v), 30 to 50%(v/v), 30 to 45%(v/v), 30 to 40%(v/v), 30 to 35%(v/v), 35 to 100%(v/v), 35 to 98%(v/v), 35 to 95%(v/v), 35 to 92%(v/v), 35 to 90%(v/v), 35 to 85%(v/v), 35 to 80%(v/v), 35 to 75%(v/v), 35 to 70%(v/v), 35 to 65%(v/v), 35 to 60%(v/v), 35 to 55%(v/v), 35 to 50%(v/v), 35 to 45%(v/v), 35 to 40%(v/v), 40 to 100%(v/v), 40 to 98%(v/v), 40 to 95%(v/v), 40 to 92%(v/v), 40 to 90%(v/v), 40 to 85%(v/v), 40 to 80%(v/v), 40 to 75%(v/v), 40 to 70%(v/v), 40 to 65%(v/v), 40 to 60%(v/v), 40 to 55%(v/v), 40 to 50%(v/v), 40 to 45%(v/v), 45 to 100%(v/v), 45 to 98%(v/v), 45 to 95%(v/v), 45 to 92%(v/v), 45 to 90%(v/v), 45 to 85%(v/v), 45 to 80%(v/v), 45 to 75%(v/v), 45 to 70%(v/v), 45 to 65%(v/v), 45 to 60%(v/v), 45 to 55%(v/v), 45 to 50%(v/v), 50 to 100%(v/v), 50 to 98%(v/v), 50 to 95%(v/v), 50 to 92%(v/v), 50 to 90%(v/v), 50 to 85%(v/v), 50 to 80%(v/v), 50 to 75%(v/v), 50 to 70%(v/v), 50 to 65%(v/v), 50 to 60%(v/v), 50 to 55%(v/v), 55 to 100%(v/v), 55 to 98%(v/v), 55 to 95%(v/v), 55 to 92%(v/v), 55 to 90%(v/v), 55 to 85%(v/v), 55 to 80%(v/v), 55 to 75%(v/v), 55 to 70%(v/v), 55 to 65%(v/v), 55 to 60%(v/v), 60 to 100%(v/v), 60 to 98%(v/v), 60 to 95%(v/v), 60 to 92%(v/v), 60 to 90%(v/v), 60 to 85%(v/v), 60 to 80%(v/v), 60 to 75%(v/v), 60 to 70%(v/v), 60 to 65%(v/v), 65 to 100%(v/v), 65 to 98%(v/v), 65 to 95%(v/v), 65 to 92%(v/v), 65 to 90%(v/v), 65 to 85%(v/v), 65 to 80%(v/v), 65 to 75%(v/v), 65 to 70%(v/v), 70 to 100%(v/v), 70 to 98%(v/v), 70 to 95%(v/v), 70 to 92%(v/v), 70 to 90%(v/v), 70 to 85%(v/v), 70 to 80%(v/v), 70 to 75%(v/v), 75 to 100%(v/v), 75 to 98%(v/v), 75 to 95%(v/v), 75 to 92%(v/v), 75 to 90%(v/v), 75 to 85%(v/v), 75 to 80%(v/v), 80 to 100%(v/v), 80 to 98%(v/v), 80 to 95%(v/v), 80 to 92%(v/v), 80 to 90%(v/v), 80 to 85%(v/v), 85 to 100%(v/v), 85 to 98%(v/v), 85 to 95%(v/v), 85 to 92%(v/v), 85 to 90%(v/v), 90 to 100%(v/v), 90 to 98%(v/v) alcohol, and/or may be obtained by extracting at 30 to 75°C, 30 to 70°C, 30 to 65°C, 30 to 60°C, 30 to 50°C, 30 to 40°C, 40 to 100°C, 40 to 90°C, 40 to 80°C, 40 to 75°C, 40 to 70°C, 40 to 65°C, 40 to 60°C, 40 to 50°C, 50 to 100°C, 50 to 90°C, 50 to 80°C, 50 to 75°C, 50 to 70°C, 50 to 65°C, 50 to 60°C, 60 to 100°C, 60 to 90°C, 60 to 80°C, 60 to 75°C, 60 to 70°C, 60 to 65°C, 65 to 100°C, 65 to 90°C, 65 to 80°C, 65 to 75°C, 65 to 70°C, 70 to 100°C, 70 to 90°C, 70 to 80°C, 70 to 75°C, 75 to 100°C, 75 to 90°C, 75 to 80°C, 80 to 100°C, 80 to 90°C or 90 to 100°C.

The avocado extract may be an extract obtained by extracting avocado with an organic solvent, for example, petroleum ether (CAS No. 101316-46-5).

The extraction hour of the extracting broccoli and/or avocado is sufficient as long as the extraction can be made sufficiently, and it may be set to about 1 hour or more, 2 hours or more, 3 hours or more, or 4 hours or more, for example, 1 to 24 hours, 2 to 24 hours, 3 to 24 hours, 4 to 24 hours, 1 to 12 hours, 2 to 12 hours, 3 to 12 hours, 4 to 12 hours, 1 to 6 hours, 2 to 6 hours, 3 to 6 hours, or 4 to 6 hours, but not limited thereto.

The extraction process to obtain the broccoli extract and/or avocado extract may be performed by all the commonly used extraction methods, and for example, it may be performed by one or more kinds of methods selected from the group consisting of hot water extraction, ultrasonic extraction, reflux extraction and the like, but not limited thereto. After the extraction process, drying and/or concentrating any extract by a common method may be further comprised.

The extraction may be performed once or twice or more (for example, twice, three times, four times or five times).

The mixing ratio of the broccoli extract and avocado extract in the mixture of the extract may be 1:0.1 to 10, 1:0.1 to 5, 1:0.1 to 4, 1:0.1 to 3, 1:0.1 to 2.5, 1:0.1 to 2, 1:0.1 to 1.5, 1:0.1 to 1, 1:0.1 to 0.67, 1:0.1 to 0.5, 1:0.1 to 0.4, 1:0.1 to 0.33, 1:0.1 to 0.25, 1:0.1 to 0.2, 1:0.2 to 10, 1:0.2 to 5, 1:0.2 to 4, 1:0.2 to 3, 1:0.2 to 2.5, 1:0.2 to 2, 1:0.2 to 1.5, 1:0.2 to 1, 1:0.2 to 0.67, 1:0.2 to 0.5, 1:0.2 to 0.4, 1:0.2 to 0.33, 1:0.2 to 0.25, 1:0.25 to 10, 1:0.25 to 5, 1:0.25 to 4, 1:0.25 to 3, 1:0.25 to 2.5, 1:0.25to 2, 1:0.25 to 1.5, 1:0.25 to 1, 1:0.25 to 0.67, 1:0.25 to 0.5, 1:0.25 to 0.4, 1:0.25 to 0.33, 1:0.33 to 10, 1:0.33 to 5, 1:0.33 to 4, 1:0.33 to 3, 1:0.33 to 2.5, 1:0.33 to 2, 1:0.33 to 1.5, 1:0.33 to 1, 1:0.33 to 0.67, 1:0.33 to 0.5, 1:0.33 to 0.4, 1:0.4 to 10, 1:0.4 to 5, 1:0.4 to 4, 1:0.4 to 3, 1:0.4 to 2.5, 1:0.4 to 2, 1:0.4 to 1.5, 1:0.4 to 1, 1:0.4 to 0.67, 1:0.4 to 0.5, 1:0.5 to 10, 1:0.5 to 5, 1:0.5 to 4, 1:0.5 to 3, 1:0.5 to 2.5, 1:0.5 to 2, 1:0.5 to 1.5, 1:0.5 to 1, or 1:0.5 to 0.67 (so far, solid weight of broccoli extract: solid weight of avocado extract), based on the solid weight. The solid weight means the weight of the solid remained after removing the solvent component of the extract. This is a term used to indicate that the mixing ratio means the ratio between the weights of active ingredients from which the extraction solvent

is removed.

The broccoli extract, avocado extract or mixture thereof described herein may be a form of an extract (crude solution), a concentrate of the extract, or a dried product of the extract or concentrate.

The extract may comprise one or more kinds selected from the group consisting of sulforaphane, indole-3-carbinol, β-sitosterol, and salts thereof.

Use

The active ingredient (compound and/or extract), composition comprising the same, and method using the same provided herein may have an effect of promoting hair growth (or hair growth promotion) and/or inhibiting (preventing, alleviating and/or treating) hair loss.

Herein, 'hair growth' refers to all actions that induce and/or promote generation and/or growth of hair, and 'hair loss' refers to all states in which hair is reduced. The hair cycle may be divided into an anagen stage in which hair grows, a catagen stage in which hair bulbs stop growing and shrink, a telogen stage in which old hair is depilated by generating new hair, and a new anagen stage in which new hair is generated as the last stage of the telogen stage. Hair may have a periodicity by repeating the above-mentioned new anagen stage, anagen stage, catagen stage, and telogen stage, and when there is a symptom of hair loss, the telogen stage may be prolonged.

Herein, 'treatment' is used in the meaning of encompassing reduction, improvement and alleviation of symptoms (hair loss), reduction of the range of symptoms, delay of symptom progression, and the like, or any one or more selected therefrom. 'Prevention' is used in the meaning of encompassing all mechanisms and/or effects that act on a subject not having symptoms (hair loss) to prevent the symptoms from appearing or to delay the onset age. "Improvement" may mean at least reducing a parameter related to the condition in which symptoms are treated by administration of the composition according to one example, for example, the severity of symptoms.

The subject of application of the composition (pharmaceutical composition, cosmetic composition and/or food composition) and/or active ingredient (active compound and/or extract) provided herein may be mammals including humans, dogs, cats, horses, cattle, pigs, goats, rabbits, mice, rats, and the like, for example, humans.

In the pharmaceutical composition or method related thereto provided herein, the administration or application method of the composition and/or active ingredient may be all methods commonly used, and for example, it may be oral administration, or parenteral administration such as dermal (for example, scalp) application, percutaneous administration (through scalp). The composition may be used by being formulated into oral formulations such as powders, granules, tablets, capsules, suspensions, emulsions, syrups, aerosols, and the like, or parenteral formulations such as suspensions, emulsions, aerosols, ointment, patches, gel, and the like, according to common methods.

The pharmaceutical composition provided herein may further contain an adjuvant such as a pharmaceutically and/or physiologically acceptable carrier, excipient and/or diluent, and the like, in addition to the active ingredient. The example of the carrier, excipient or diluent may include one or more kinds selected from the group consisting of lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil, and the like. When formulated, one or more kinds of diluents or excipients selected from the group consisting of commonly used fillers, thickeners, binders, wetting agents, disintegrating agents, surfactants and the like may be used. The solid formulation for oral administration comprises one or more kinds selected from the group consisting of tablets, pills, powders, granules, capsules, syrups, powders, suspensions, and the like, and this solid formulation may be prepared by mixing at least one or more excipients, for example, one or more kinds selected from the group consisting of starch, calcium carbonate, sucrose, lactose, gelatin, and the like, to the extract. In addition, lubricants such as magnesium stearate talc are used in addition to the simple excipient. One or more kinds selected from the group consisting of suspensions, oral liquids, emulsions, syrups, and the like correspond to the liquid formulation for oral administration, and in addition to the commonly used simple diluent, water or liquid paraffin, various excipients, for example, one or more kinds selected from the group consisting of wetting agents, sweeteners, aromatic agents, preservatives, and the like may be comprised. The formulation for parenteral administration includes one or more kinds selected from the group consisting of sterile aqueous solutions, non-aqueous solutions, suspensions, emulsions, freeze-dried preparations, transdermal preparations, and the like. As the non-aqueous solutions or suspensions, one or more kinds selected from the group consisting of propylene glycol, polyethylene glycol, plant oil such as olive oil, injectable ester such as ethyl oleate may be used.

The content of the active ingredient (active compound (sulforaphane, indole-3-carbinol, β-sitosterol, or a combination thereof) and/or extract (solid)) contained in the pharmaceutical composition may be suitably determined according to the form of the formulation, desired effect, and the like, and for example, it may be 0.0001 to 99 % by weight, 0.0001 to 90 % by weight, 0.0001 to 80 % by weight, 0.0001 to 70 % by weight, 0.0001 to 60 % by weight, 0.0001 to 50 % by weight, 0.0001 to 40 % by weight, 0.0001 to 30 % by weight, 0.0001 to 20 % by weight, 0.0001 to 10 % by weight, 0.01 to 99 % by weight, 0.01 to 90 % by weight, 0.01 to 80 % by weight, 0.01 to 70 % by weight, 0.01 to 60 % by weight,

0.01 to 50 % by weight, 0.01 to 40 % by weight, 0.01 to 30 % by weight, 0.01 to 20 % by weight, 0.01 to 10 % by weight, 1 to 99 % by weight, 1 to 90 % by weight, 1 to 80 % by weight, 1 to 70 % by weight, 1 to 60 % by weight, 1 to 50 % by weight, 1 to 40 % by weight, 1 to 30 % by weight, 1 to 20 % by weight, 1 to 10 % by weight, 5 to 99 % by weight, 5 to 90 % by weight, 5 to 80 % by weight, 5 to 70 % by weight, 5 to 60 % by weight, 5 to 50 % by weight, 5 to 40 % by weight, 5 to 30 % by weight, 5 to 20 % by weight, 5 to 10 % by weight, 10 to 99 % by weight, 10 to 90 % by weight, 10 to 80 % by weight, 10 to 70 % by weight, 10 to 60 % by weight, 10 to 50 % by weight, 10 to 40 % by weight, 10 to 30 % by weight, 10 to 20 % by weight, 20 to 99 % by weight, 20 to 90 % by weight, 20 to 80 % by weight, 20 to 70 % by weight, 20 to 60 % by weight, 20 to 50 % by weight, 20 to 40 % by weight, 20 to 30 % by weight, 30 to 99 % by weight, 30 to 90 % by weight, 30 to 80 % by weight, 30 to 70 % by weight, 30 to 60 % by weight, 30 to 50 % by weight, 30 to 40 % by weight, 40 to 99 % by weight, 40 to 90 % by weight, 40 to 80 % by weight, 40 to 70 % by weight, 40 to 60 % by weight, 40 to 50 % by weight, 50 to 99 % by weight, 50 to 90 % by weight, 50 to 80 % by weight, 50 to 70 % by weight, 50 to 60 % by weight, 60 to 99 % by weight, 60 to 90 % by weight, 60 to 80 % by weight, or 60 to 70 % by weight of the total weight of the pharmaceutical composition.

The cosmetic composition provided herein may further comprise commonly used components for cosmetic compositions, in addition to the active ingredient, and for example, one or more kinds selected from the group consisting of common adjuvants such as anti-oxidants, stabilizers, solubilizers, vitamins, pigments and flavorings, and carriers may be further comprised. As the carrier, one or more kinds selected from the group consisting of purified water, monohydric alcohols (ethanol or propyl alcohol), polyhydric alcohols (glycerol, 1,3-butylene glycol or propylene glycol), higher fatty acids (palmitic acid or linolenic acid), fat and oils (wheat germ oil, camellia oil, jojoba oil, olive oil, squalene, sunflower oil, macademia peanut oil, avocado oil or fatty acid glyceride), and the like may be used, but not limited thereto. In addition, if necessary, one or more kinds selected from the group consisting of surfactants, moisturizing agents, preservatives, anti-oxidants, and the like may be added.

The formulation of the cosmetic composition may be all forms applied to hair, and for example, it may be one or more selected from the group consisting of hair products such as solutions, sprays, emulsions, gel, cream, essence, packs, ample, lotion, shampoo, soaps, oil and the like.

The content of the active ingredient (active compound (sulforaphane, indole-3-carbinol, β-sitosterol, or a combination thereof) and/or extract (solid)) contained in the cosmetic composition may be suitably determined according to the form of the formulation, desired effect, and the like, and for example, it may be 0.0001 to 99 % by weight, 0.0001 to 90 % by weight, 0.0001 to 80 % by weight, 0.0001 to 70 % by weight, 0.0001 to 60 % by weight, 0.0001 to 50 % by weight, 0.0001 to 40 % by weight, 0.0001 to 30 % by weight, 0.0001 to 20 % by weight, 0.0001 to 10 % by weight, 0.01 to 99 % by weight, 0.01 to 90 % by weight, 0.01 to 80 % by weight, 0.01 to 70 % by weight, 0.01 to 60 % by weight, 0.01 to 50 % by weight, 0.01 to 40 % by weight, 0.01 to 30 % by weight, 0.01 to 20 % by weight, 0.01 to 10 % by weight, 1 to 99 % by weight, 1 to 90 % by weight, 1 to 80 % by weight, 1 to 70 % by weight, 1 to 60 % by weight, 1 to 50 % by weight, 1 to 40 % by weight, 1 to 30 % by weight, 1 to 20 % by weight, 1 to 10 % by weight, 5 to 99 % by weight, 5 to 90 % by weight, 5 to 80 % by weight, 5 to 70 % by weight, 5 to 60 % by weight, 5 to 50 % by weight, 5 to 40 % by weight, 5 to 30 % by weight, 5 to 20 % by weight, 5 to 10 % by weight, 10 to 99 % by weight, 10 to 90 % by weight, 10 to 80 % by weight, 10 to 70 % by weight, 10 to 60 % by weight, 10 to 50 % by weight, 10 to 40 % by weight, 10 to 30 % by weight, 10 to 20 % by weight, 20 to 99 % by weight, 20 to 90 % by weight, 20 to 80 % by weight, 20 to 70 % by weight, 20 to 60 % by weight, 20 to 50 % by weight, 20 to 40 % by weight, 20 to 30 % by weight, 30 to 99 % by weight, 30 to 90 % by weight, 30 to 80 % by weight, 30 to 70 % by weight, 30 to 60 % by weight, 30 to 50 % by weight, 30 to 40 % by weight, 40 to 99 % by weight, 40 to 90 % by weight, 40 to 80 % by weight, 40 to 70 % by weight, 40 to 60 % by weight, 40 to 50 % by weight, 50 to 99 % by weight, 50 to 90 % by weight, 50 to 80 % by weight, 50 to 70 % by weight, 50 to 60 % by weight, 60 to 99 % by weight, 60 to 90 % by weight, 60 to 80 % by weight, or 60 to 70 % by weight of the total weight of the cosmetic composition.

The health functional food is a food manufactured using raw materials or ingredients (hereinafter, 'functional raw materials') that are easily deficient in daily meals or have useful functions for the human body, and refers to any food that helps to maintain health or prevent and/or improve certain diseases or symptoms, and there are no special limitations on the form of the final product. For example, the health functional food may be selected from the group consisting of various kinds of foods, beverages, food additives, and the like, but not limited thereto.

The content of the active ingredient (active compound (sulforaphane, indole-3-carbinol, β-sitosterol, or a combination thereof) and/or extract (solid)) contained in the food composition (health functional food) may be suitably determined according to the form of the formulation, desired effect, and the like, and for example, it may be 0.0001 to 99 % by weight, 0.0001 to 90 % by weight, 0.0001 to 80 % by weight, 0.0001 to 70 % by weight, 0.0001 to 60 % by weight, 0.0001 to 50 % by weight, 0.0001 to 40 % by weight, 0.0001 to 30 % by weight, 0.0001 to 20 % by weight, 0.0001 to 10 % by weight, 0.01 to 99 % by weight, 0.01 to 90 % by weight, 0.01 to 80 % by weight, 0.01 to 70 % by weight, 0.01 to 60 % by weight, 0.01 to 50 % by weight, 0.01 to 40 % by weight, 0.01 to 30 % by weight, 0.01 to 20 % by weight, 0.01 to 10 % by weight, 1 to 99 % by weight, 1 to 90 % by weight, 1 to 80 % by weight, 1 to 70 % by weight, 1 to 60 % by weight, 1 to 50 % by weight, 1 to 40 % by weight, 1 to 30 % by weight, 1 to 20 % by weight, 1 to 10 % by weight, 5 to

99 % by weight, 5 to 90 % by weight, 5 to 80 % by weight, 5 to 70 % by weight, 5 to 60 % by weight, 5 to 50 % by weight, 5 to 40 % by weight, 5 to 30 % by weight, 5 to 20 % by weight, 5 to 10 % by weight, 10 to 99 % by weight, 10 to 90 % by weight, 10 to 80 % by weight, 10 to 70 % by weight, 10 to 60 % by weight, 10 to 50 % by weight, 10 to 40 % by weight, 10 to 30 % by weight, 10 to 20 % by weight, 20 to 99 % by weight, 20 to 90 % by weight, 20 to 80 % by weight, 20 to 70 % by weight, 20 to 60 % by weight, 20 to 50 % by weight, 20 to 40 % by weight, 20 to 30 % by weight, 30 to 99 % by weight, 30 to 90 % by weight, 30 to 80 % by weight, 30 to 70 % by weight, 30 to 60 % by weight, 30 to 50 % by weight, 30 to 40 % by weight, 40 to 99 % by weight, 40 to 90 % by weight, 40 to 80 % by weight, 40 to 70 % by weight, 40 to 60 % by weight, 40 to 50 % by weight, 50 to 99 % by weight, 50 to 90 % by weight, 50 to 80 % by weight, 50 to 70 % by weight, 50 to 60 % by weight, 60 to 99 % by weight, 60 to 90 % by weight, 60 to 80 % by weight, or 60 to 70 % by weight of the total weight of the pharmaceutical composition.

The health functional food may further contain one or more kinds selected from the group consisting of various nutrients, vitamins, minerals (electrolytes), flavoring agents such as synthetic flavoring agents or natural flavoring agents, coloring agents, improving agents (cheese, chocolate, etc.), pectic acids or salts thereof, alginic acids or salts thereof, organic acids, protective colloidal thickeners, pH adjusting agents, stabilizers, preservatives, glycerin, alcohols, carbonating agents used for carbonated beverages, and the like. The ratio of this additive is generally selected in the range of about 0.00001 to about 20 parts by weight based on 100 parts by weight of the total health functional food, but not limited thereto.

The composition and/or active ingredient (active compound and/or extract) provided herein may be administered in a pharmaceutically effective dose. Herein, "pharmaceutically effective dose" or "effective dose" may refer to a dose to contributes to the desired effect (promoting hair growth and/or inhibiting hair loss), or exhibit a beneficial action for the effect, or achieve the effect. The pharmaceutically effective dose may be prescribed in various ways depending on factors such as formulation method, application subject's age, weight, gender, symptom level, administration hour, administration interval, excretion rate, reaction sensitivity and the like. The effective dose may vary depending on the subject's age, body weight, gender, administration form, health condition, symptom level, and the like, and it may be administered once a day to several times in divided at a regular hour interval according to judgment of a doctor or pharmacist.

For example, a single or daily dose of the composition may be in the range of 0.0001 to 10000mg/kg, specifically, 0.001 to 1000mg/kg, 0.001 to 500mg/kg, 0.001 to 300mg/kg, 0.001 to 250mg/kg, 0.01 to 1000mg/kg, 0.01 to 500mg/kg, 0.01 to 300mg/kg, 0.01 to 250mg/kg, 0.1 to 1000mg/kg, 0.1 to 500mg/kg, 0.1 to 300mg/kg, 0.1 to 250mg/kg, 1 to 1000mg/kg, 1 to 500mg/kg, 1 to 300mg/kg, 1 to 250mg/kg, 10 to 1000mg/kg, 10 to 500mg/kg, 10 to 300mg/kg, or 10 to 250mg/kg, based on the weight of the active ingredient (active compound (sulforaphane, indole-3-carbinol, β-sitosterol, or a combination thereof) and/or extract (solid)), but not limited thereto. The single or daily dose may be formulated into one formulation as a unit dosage form, or formulated by appropriately portioning, or prepared by internalizing in a multi-dose container. The dose is an example of an average case, and the dose may be higher or lower depending on individual differences.

[ADVANTAGEOUS EFFECTS]

The present specification provides a composition for promoting hair growth or inhibiting hair loss with excellent biosafety and with an excellent effect of promoting hair growth or inhibiting hair loss, by comprising a broccoli *(Brassica oleracea* var. italica) extract, an avocado *(Persea americana)* extract, and a mixture thereof, or an effective compound derived from the natural extracts.

[BRIEF DESCRIPTION OF THE DRAWINGS]

FIG. 1a is a graph showing the testosterone concentration in serum of the rat administered with the broccoli extract, avocado extract or mixture thereof according to one example.

FIG. 1b is a graph showing the dihydrotestosterone (DHT) concentration in serum of the rat administered with the broccoli extract, avocado extract or mixture thereof according to one example.

FIG. 2 is photographs of staining the skin tissue of the rat administered with the broccoli extract, avocado extract or mixture thereof according to one example.

FIG. 3 is a graph showing the length of the hair follicle in the skin of the rat administered with the broccoli extract, avocado extract or mixture thereof according to one example.

FIG. 4a is a graph showing the testosterone concentration in serum of the rat administered with sulforaphane, indole-3-carbionol and beta-sitosterol each or in combination.

FIG. 4b is a graph showing the dihydrotestosterone concentration in serum of the rat administered with sulforaphane,

indole-3-carbionol and beta-sitosterol each or in combination.
FIG. 5 is a graph showing the length of the hair follicle in the skin of the rat administered with sulforaphane, indole-3-carbionol and beta-sitosterol each or in combination.

[MODE FOR INVENTION]

Hereinafter, the present invention will be described in more detail by the following examples. However, they are intended to illustrate the present invention only, but the scope of the present invention is not limited by these examples.

**Example 1: Preparation of extract**

**1.1. Preparation of broccoli extract**

Broccoli (*Brassica oleracea* var. italica) was washed using clean water and then dried sufficiently. To the powder 100g obtained by crushing the dried broccoli, 10 volume times (500ml) of water or ethanol (10 to 100%(v/v) ethanol (spirit)) was added, and after extracting at 70°C for 3 hours or more three times, the filtrate filtered through a 1$\mu$m (micrometer) filter was concentrated with heating until it reached 10%(w/w) of the original weight. The obtained concentrate was continuously concentrated, dried completely, and then powdered to prepare a broccoli extract powder. Among them, broccoli water extract powder (hereinafter, referred to as BE) using water as an extraction solvent was used for subsequent tests.

**1.2. Preparation of avocado extract**

Avocado (*Persea americana*) was sterilized in chlorinated water for 15 minutes. The avocado flesh was separated and homogenized after adding a 1%(v/v) citric acid solution. The homogenized solution was lyophilized and powdered and stored frozen for 1 week before extraction. The prepared freeze-dried powder 100g was dissolved in 500mL petroleum ether (CAS No. 101316-46-5) and extracted with a Soxhlet extractor to obtain an oily extract. The extracted oil was powdered to prepare an avocado extract powder (hereinafter, referred to as AE).

**Example 2: Confirmation of male hormone reducing effect of broccoli extract and/or avocado extract**

The effect of reducing testosterone and dihydrotestosterone (hereinafter, referred to as DHT) of the broccoli extract and/or avocado extract prepared in Example 1 was confirmed by collecting plasma of male wistar albino rats and using a testosterone and DHT ELISA kit.

**Example 2-1. Breeding of experimental animals**

Male wistar albino rats weight 180 to 240 g, February to March age were bred under the conditions of a constant light-dark cycle in which light was applied from 8 am to 8 pm, a temperature of 22 to 25 °C, and a relative humidity of 60%. For the feed of experimental animals, a general pellet dry feed was used, and feed and water were allowed to be consumed at all times.

**Example 2-2. Measurement of concentration of testosterone and DHT after extract administration**

To the experimental animals bred in Example 2-1, testosterone solution 0.2mℓ obtained by diluting 100 mg of commercially available testosterone (SUSTANON®) in arachis oil 100mℓ was subcutaneously injected daily for a total of 21 days.
In addition, the broccoli extract and/or avocado extract prepared in Example 1 were orally administered for a total of 21 days daily so as to be a dose of 100mg per 1 kg of the body weight of each experimental animal (in case of coadministration group, broccoli extract 67mg/kg + avocado extract 33mg/kg).
The experimental animal groups treated with the testosterone solution, broccoli extract, avocado extract or mixture of broccoli extract and avocado extract were arranged in Table 1 below.

[Table 1]

| | Testosterone (T; for 21 days) | Broccoli extract (BE 100mg/kg) | Avocado extract (AE 100mg/kg) | Extract mixture (BE 67mg/kg +AE 33mg/kg) |
|---|---|---|---|---|
| Normal group (Group 1) | - | - | - | - |
| Negative control group (Group 2) | ○ | | | |
| Experimental group (Group 3) | ○ | ○ | | |
| Experimental group (Group 4) | ○ | | ○ | |
| Experimental group (Group 5) | ○ | | | ○ |

For 21 days, the testosterone solution and broccoli extract, avocado extract or broccoli and avocado extract were administered, and on the next day, before skin samples were collected, plasma from rats was extracted and the concentration of testosterone and DHT were measured using a testosterone and DHT ELISA kit (Arigo Biolaboratories, Taiwan) according to the manufacturer's instructions.

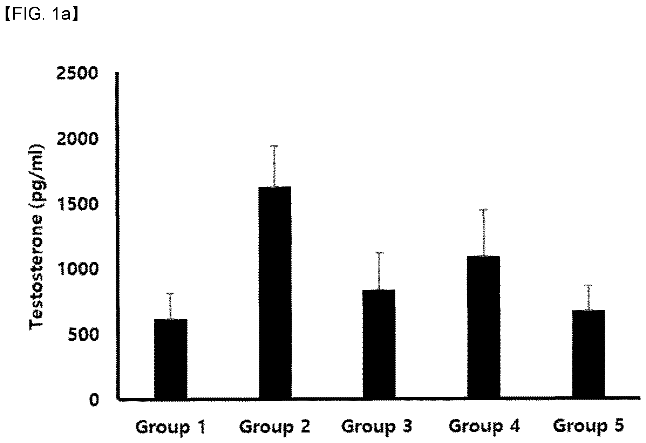

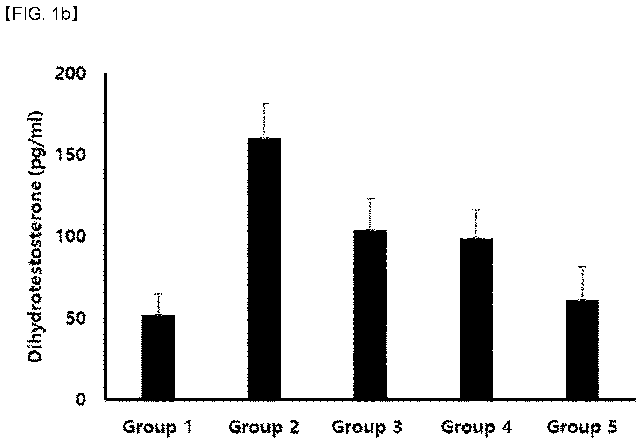

The result of measuring the testosterone and DHT in plasma of the normal group, negative control group and experimental groups was shown in FIG. 1a and FIG. 1b, respectively. As shown in FIG. 1a and FIG. 1b, in case of the negative control group administered only with the testosterone solution, the concentration of testosterone and DHT in plasma was largely increased, compared to the normal group. On the other hand, in case of experimental groups administered with the broccoli extract and/or avocado extract (Groups 3, 4, 5), the concentration of testosterone and DHT in plasma was reduced, and among them, in particular, in case of the experimental group administered with the mixture of the broccoli extract and avocado extract (Group 5), the concentration of testosterone and DHT in plasma was significantly reduced at a level equal to the normal group (Group 1).

**Example 3: Confirmation of effect on hair follicles of broccoli extract or avocado extract**

**Example 3-1. Shape change of hair follicles (stained photograph)**

By staining the skin tissue of experimental animals, the shape change of hair follicles was investigated. To the test animals prepared in Example 2-1, the testosterone solution and broccoli extract, avocado extract or mixture thereof was administered for 21 days, and on the next day, the skin of the dorsal area of experimental animals was removed using a hair removing drug (Hair removing cream Veet®), and then it was incised and fixed with 10%(v/v) formalin. After 24 hours, the skin sample was soaked in paraffin wax and sectioned to a uniform thickness of 10$\mu$m, and hair follicles were stained with haematoxylin and eosin. Slides were observed under a microscope and images were recorded using standard software (Leica, Germany).

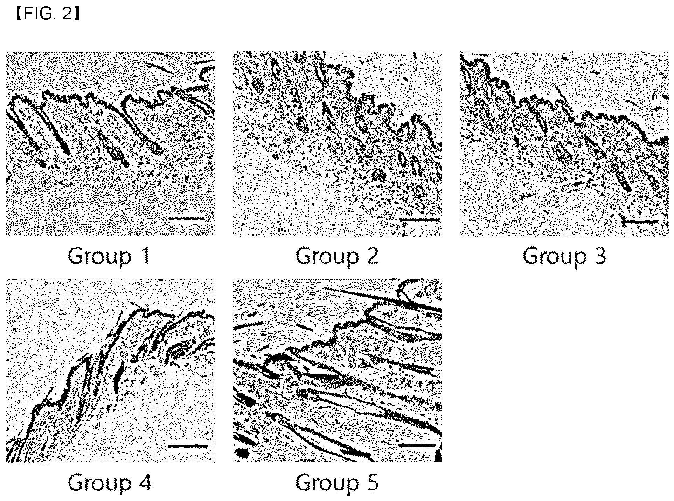

The stained photograph of the skin tissue of the observed normal group, negative control group and experimental groups was shown in FIG. 2. As shown in FIG. 2, in case of the negative control group administered only with the testosterone solution, many hair follicles in the telogen stage were found. On the other hand, in case of the experimental groups (Groups 3, 4, 5) administered with the broccoli extract and/or avocado extract, the length of hair follicles was increased, and the length of hair follicles was found to be similar to that of the normal group (Group 1).

**Example 3-2. Measurement of density of hair follicles**

The density of hair follicles was measured in the skin of the normal group, negative control group and experimental group and shown in Table 2 below. The density of hair follicles was measured by counting the number of hair follicles in a unit area (1mm$^2$). The obtained result was shown in Table 2.

[Table 2]

| | Hair follicle density | Rate of change compared to normal group |
|---|---|---|
| Normal group (Group 1) | 1.31±0.021 | - |

(continued)

| | Hair follicle density | Rate of change compared to normal group |
|---|---|---|
| Negative control group (Group 2) | 0.91±0.069 | -30.53% |
| Experimental group (Group 3) | 2.40±0.053* | +83.21% |
| Experimental group (Group 4) | 2.09±0.032* | +59.54% |
| Experimental group (Group 5) | 2.61±0.057* | +99.24% |
| (In Table 2, Values are mean ± SEM, n = 15, *p<0.05 as compared to negative control) | | |

The rate of change compared to the normal group described in Table 2 was calculated according to Equation 1 below.

(Equation 1)

Rate of change compared to normal group = (hair follicle density of negative control group or experimental group – hair follicle density of normal group) / hair follicle density of normal group

As shown in Table 2 above, in case of the negative control group administered only with the testosterone solution, the hair follicle density was reduced by about 31% compared to the normal group. On the other hand, in case of the experimental groups (Groups 3, 4, 5) administered with the broccoli extract and/or avocado extract, they exhibited an excellent effect that the hair follicle density was increased by about 83% (broccoli extract), about 60% (avocado extract) or about 99% (mixture of broccoli extract and avocado extract), respectively, compared to the normal group.

**Example 3-3. Comparison of condition of hair follicles**

The ratio of the anagen stage and telogen stage of hair follicles in the skin of the normal group, negative control group and experimental groups was calculated and shown in Table 3 below.

[Table 3]

| | Anagen | Telogen | Anagen/telogen ratio | Rate of change compared to normal group |
|---|---|---|---|---|
| Normal group (Group 1) | 66.1 | 33.9 | 1 .949853 | - |
| Negative control group (Group 2) | 14.6 | 85.4 | 0.17096 | -91.23% |
| Experimental group (Group 3) | 60.4 | 39.6 | 1.525253* | -21.78% |
| Experimental group (Group 4) | 51.2 | 48.8 | 1.04918* | -46.19% |
| Experimental group (Group 5) | 65.6 | 34.4 | 1.906977* | -2.20% |
| *p<0.05 as compared to negative control | | | | |

The rate of change compared to the normal group in Table 3 was calculated according to Equation 2 below.

(Equation 2)

Rate of change compared to normal group = (anagen/telogen ratio of negative control group or experimental group – anagen/telogen ratio of normal group) / anagen/telogen ratio of normal group

As shown in Table 3, in case of the negative control group administered only with the testosterone solution, the hair follicles in the anagen stage were reduced and the hair follicles in the telogen stage were increased, and therefore, the anagen/telogen ratio was reduced by about 91% compared to the normal group. On the other hand, in case of all the experimental groups (Groups 3, 4, 5) administered with the broccoli extract and/or avocado extract, compared to the negative control group, the hair follicles in the anagen stage were increased and the hair follicles of telogen stage were reduced. Among them, in particular, in case of the experimental group administered with the mixture of the broccoli extract and avocado extract (Group 5), compared to the negative control group, the hair follicles in the anagen stage were largely increased and the hair follicles in the telogen stage were significantly reduced, and the anagen/telogen ratio was shown at a similar level to the normal group.

**Example 3-4. Comparison of length of hair follicles**

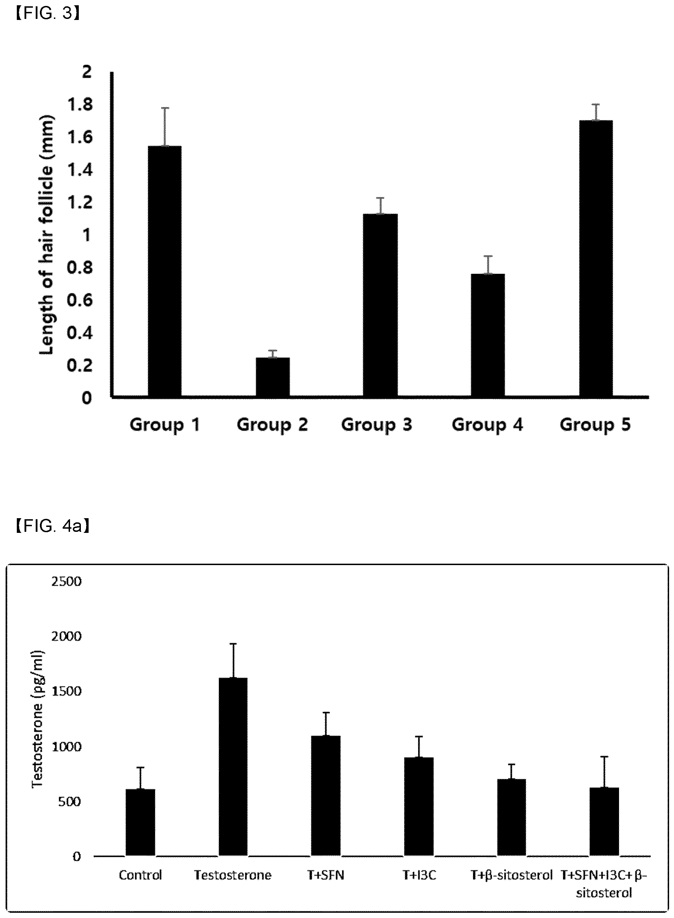

The length of hair follicles was measured in the skin of the normal group, negative control group and experimental groups and shown in FIG. 3. For the length of hair follicles, the length of stained hair follicles photographed in Example 3-1 was measured using a standard software (Leica, Germany), and the result was shown in FIG. 3.

As shown in FIG. 3, in case of the negative control group administered only with the testosterone solution, the length of hair follicles was largely reduced, compared to the normal group. On the other hand, in case of the experimental groups administered the broccoli extract and/or avocado extract (Groups 3, 4, 5), compared to the negative control group, the length of hair follicles was increased. Among them, in particular, in case of the experimental group administered with the mixture of the broccoli extract and avocado extract, compared to the negative control group, the length of hair follicles was significantly increased, and it exhibited an excellent effect that the length of hair follicles was increased, compared to the normal group.

**Example 3-5. Confirmation of effect depending on broccoli extraction solvent**

A broccoli water extract which broccoli was extracted with water according to Example 1-1 was obtained. As a comparative group, a broccoli extract was obtained in the same manner as Example 1-1, except that the extraction solvent was extracted with an aqueous ethanol solution of various concentrations (10%, 30%, 50%, 70%, 100%).

The sulforaphane (SFN) content in the broccoli water extract (DW) and comparative group (EtOH; ethanol extracts at various concentrations) was measured, and the result was shown in Table 4 below.

[Table 4]

| Extraction solvent | SFN content (ug/ml) |
|---|---|
| 100% EtOH | ND |
| 70% EtOH | ND |
| 50% EtOH | ND |
| 30% EtOH | 1.7 |
| 10% EtOH | 5.2 |
| DW | 61.41 |
| (ND: not detected) | |

As shown in Table 4 above, it was confirmed that all the extracts obtained by extracting broccoli with water or aqueous ethanol solution at various concentrations (30% and 10%) contained sulforaphane. Among them, it was confirmed that the sulforaphane content of the extract obtained by extracting broccoli with water was particularly high.

By the same method as Example 3-2, the broccoli water extract and broccoli ethanol extract were administered to the experimental animals prepared in Example 2-1 and the density of hair follicles in the skin of the experimental animals was measured and the result was shown in Table 5 below. The normal control group (control; normal group) is a group in which neither testosterone nor the extract is administered, and the negative control group (Testosterone; T) is a group in which only testosterone is administered.

[Table 5]

| Extraction solvent | Hair follicle density |
|---|---|
| Control | 1.25±0.39 |
| Testosterone | 0.98±0.23 |
| T+70% EtOH | 0.99±0.48 |
| T+30% EtOH | 1.0±0.65 |
| T+10% EtOH | 1.14±0.73 |
| T+DW | 1.93±0.102 |

The hair follicle density was shown as mean ± mean standard error. The rate of change compared to the normal group was calculated by the same method as Equation 1 above.

As shown in Table 5 above, it was confirmed that the hair follicle density reduced by testosterone was increased again, when the extract obtained by extracting broccoli with water or aqueous ethanol solution at various concentrations, compared to the negative control group administered only with the testosterone solution. In particular, it was confirmed that the hair follicle density was more significantly increased, when the extract obtained by extracting broccoli with water.

**Example 4: Confirmation of male hormone reducing effect of compounds**

To the experimental animals bred in Example 2-1, testosterone solution 0.2mℓ obtained by diluting 100 mg of commercially available testosterone (SUSTANON®) in arachis oil 100mℓ was subcutaneously injected daily for a total of 21 days, and according to Table 6 below, sulforaphane, indole-3-carbinol, beta-sitosterol or a mixture of the compounds was orally administered daily for 21 days at a dose of 10mg /kg.

The experimental animal groups treated with the sulforaphane, indole-3-carbinol, beta-sitosterol or mixture of the compounds were arranged in Table 6 below.

[Table 6]

| | Testosterone (T; for 21 days) | Sulforaphane (SFN 10mg/ kg) | Indole-3-carbinol (I3C 10mg/ kg) | β-sitosterol (10mg/kg) | Mixture of compounds (SFN 8mg/kg +I3C 1 mg/kg +β-sitosterol 1 mg/kg) |
|---|---|---|---|---|---|
| Normal group (Group A) | - | - | - | - | - |
| Negative control group (Group B) | ○ | | | | |
| Experimental group (Group C) | ○ | ○ | | | |
| Experimental group (Group D) | ○ | | ○ | | |
| Experimental group (Group E) | ○ | | | ○ | |

(continued)

| | Testosterone (T; for 21 days) | Sulforaphane (SFN 10mg/ kg) | Indole-3-carbinol (I3C 10mg/ kg) | β-sitosterol (10mg/kg) | Mixture of compounds (SFN 8mg/kg +I3C 1 mg/kg +β-sitosterol 1 mg/kg) |
|---|---|---|---|---|---|
| Experimental group (Group F) | ○ | | | | ○ |

For 21 days, sulforaphane, indole-3-carbinol, β-sitosterol or a mixture thereof was administered, and on the next day, plasma from rats was extracted and the concentration of testosterone and DHT were measured using a testosterone and DHT ELISA kit (Arigo Biolaboratories, Taiwan) (See Example 2-2).

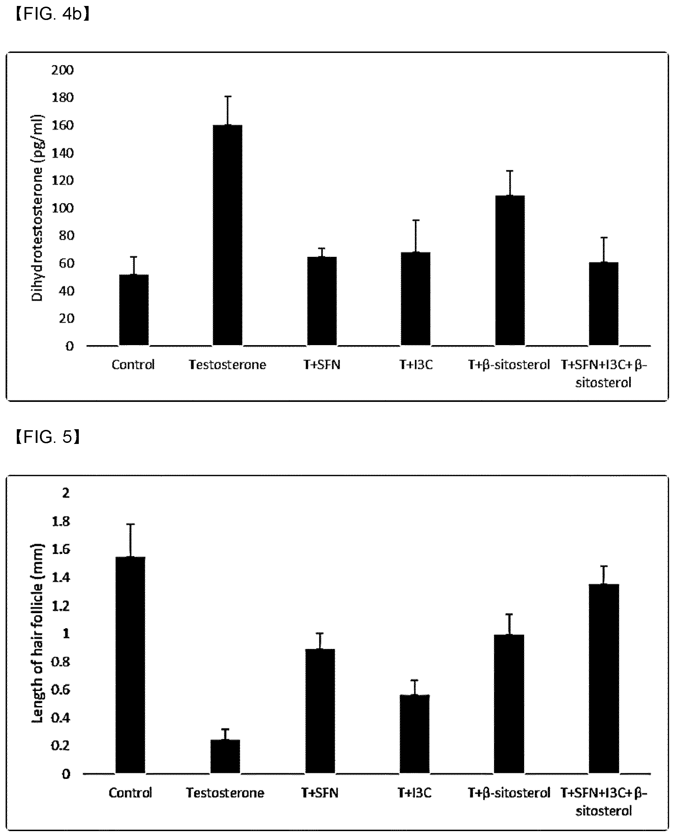

The result of measuring the concentration of testosterone and DHT in plasma of the normal group (control), negative control group and experimental group was shown in FIG. 4a (testosterone) and FIG. 4b (DHT), respectively. As shown in FIG. 4a and FIG. 4b, in case of the negative control group administered only with the testosterone solution, the concentration of testosterone and DHT in plasma was largely increased, compared to the normal group. On the other hand, it could be seen that the concentration of testosterone and DHT in plasma was significantly reduced, in case of the experimental groups administered with sulforaphane, indole-3-carbinol and/or β-sitosterol (Groups C, D, E, F). Among then, in particular, in case of the experimental group administered with the mixture of mixing sulforaphane, indole-3-carbinol and β-sitosterol (Group F), the concentration of testosterone and DHT in plasma was largely reduced at the same level as the normal group (Group A).

### Example 5: Confirmation of effect on hair follicles of compounds

### Example 5-1. Measurement of density of hair follicles

Referring to Example 3-2, the density of hair follicles was measured in the skin of the normal group, negative control group and experimental groups and shown in Table 7 below.

[Table 7]

| | Density of hair follicles | Rate of change compared to normal group (%) |
|---|---|---|
| Normal group (Group A) | 1.31±0.021 | - |
| Negative control group (Group B) | 0.91±0.069 | -30.53% |
| Experimental group (Group C) | 1.93±0.102* | +47.33% |
| Experimental group (Group D) | 1.59±0.74* | +21.37% |
| Experimental group (Group E) | 2.195±0.093* | +67.56% |
| Experimental group (Group F) | 2.41±0.064* | +83.97% |
| Values are mean ± SEM, n = 15, *p<0.05 as compared to negative control | | |

The density of hair follicles was shown as mean ± mean standard error. The rate of change compared to the normal group was calculated by the same method as Equation 1 above.

As shown in Table 7 above, in case of the negative control group administered only with the testosterone solution, the density of hair follicles was reduced by about 31% compared to the normal group. On the other hand, the experimental groups administered with sulforaphane, indole-3-carbinol, β-sitosterol or a mixture thereof (Groups C, D, E, F) exhibited an excellent effect of increasing the density of hair follicles by about 47% (sulforaphane), about 21% (indole-3-carbinol), about 68% (β-sitosterol) or about 84% (mixture of sulforaphane, indole-3-carbinol and β-sitosterol), respectively, compared to the normal group.

### Example 5-2. Comparison of condition of hair follicles

Referring to Example 3-3, the ratio of the anagen stage and telogen stage of hair follicles was calculated in the skin of the normal group, negative control group and experimental groups and shown in Table 8 below.

[Table 8]

| | Anagen | Telogen | Anagen/telogen ratio | Rate of change compared to normal group |
|---|---|---|---|---|
| Normal group (Group A) | 66.1 | 33.9 | 1.94 | - |
| Negative control group (Group B) | 14.6 | 85.4 | 0.17 | -91.23% |
| Experimental group (Group C) | 50.8 | 49.2 | 1.03* | -47.05% |
| Experimental group (Group D) | 46.8 | 53.2 | 0.87* | -54.88% |
| Experimental group (Group E) | 50.2 | 49.8 | 1.00* | -48.30% |
| Experimental group (Group F) | 58.4 | 41.6 | 1.40* | -28.00% |
| *p<0.05 as compared to negative control | | | | |

The rate of change compared to the normal group was calculated by the same method as Equation 2.

As shown in Table 8 above, in case of the negative control group administered only with the testosterone solution, the hair follicles in the anagen stage were reduced and the hair follicles in the telogen stage were increased, and the anagen/telogen ratio was reduced by about 91% compared to the normal group. On the other hand, in case of the experimental groups administered with sulforaphane, indole-3-carbinol, β-sitosterol or a mixture thereof (Groups C, D, E, F), the hair follicles in the anagen stage were increased and the hair follicles in the telogen stage were reduced, compared to the negative control group. Among them, in particular, it could be confirmed that in case of the experimental group administered with the mixture of mixing testosterone and sulforaphane, indole-3-carbinol and β-sitosterol (Group 6), the hair follicles in the anagen stage were largely increased and the hair follicles in the telogen stage were significantly reduced, compared to the negative control group.

**Example 5-3. Comparison of length of hair follicles**

Referring to Example 3-4, the length of the hair follicles was measured in the skin of the normal group, negative control group and experimental groups and shown in FIG. 5. As shown in FIG. 5, in case of the negative control group administered only with the testosterone solution, the length of hair follicles was largely reduced, compared to the normal group (control). On the other hand, in case of the experimental groups administered with sulforaphane, indole-3-carbinol, β-sitosterol or a mixture thereof (Groups C, D, E, F), the length of hair follicles was increased, compared to the negative control group. Among them, in particular, it could be confirmed that in case of the experimental group administered with the mixture of mixing testosterone and sulforaphane, indole-3-carbinol and β-sitosterol (Group F), the length of hair follicles was largely increased, compared to the negative control group.

**Example 5-4. Confirmation of effect according to mixing ratio of compounds**

To the experimental animals bred in Example 2-1, mixtures of mixing sulforaphane, indole-3-carbinol and β-sitosterol at various mixing ratios as Table 9 below by the same method as Example 4 were orally administered daily at a dose of 10mg/kg in total for 21 days. The normal control group (control; normal group) is a group in which neither testosterone nor the extract is administered, and the negative control group (Testosterone; T) is a group in which only testosterone is administered.

Referring to Example 3-2, the density of hair follicles was measured in the skin of the normal control group, negative control group and experimental groups and shown in Table 9 below.

[Table 9]

| Mixing ratio | Density of hair follicles | Rate of change compared to normal group |
|---|---|---|
| Control | 1.25±0.39 | - |
| Testosterone | 0.98±0.23 | -21.6% |
| T+8:1:1 (SFN 8mg/kg + I3C 1mg/kg + S1mg/kg) | 2.41±0.064 | +92.80% |

(continued)

| Mixing ratio | Density of hair follicles | Rate of change compared to normal group |
|---|---|---|
| T+4:3:3 (SFN 4mg/kg + I3C 3mg/kg + S 3mg/kg) | 1.96±0.031 | +56.80% |
| T+1:8:1 (SFN 1mg/kg + I3C 8mg/kg + S 1mg/kg) | 1.60±0.039 | +28.00% |
| T+3:3:4 (SFN 3mg/kg + I3C 3mg/kg + S 4mg/kg) | 1.91 ±0.052 | +52.80% |
| T+1:1:8 (SFN 1mg/kg + I3C 1mg/kg + S 8mg/kg) | 1.87±0.071 | +49.60% |
| (SFN: sulforaphane, I3C: indole-3-carbinol, S: beta-sitosterol) | | |

The density of hair follicles was shown as mean ± mean standard error. The rate of change compared to the normal group was calculated by the same method as Equation 1 above.

As shown in Table 9 above, in case of the negative control group administered only with the testosterone solution, the density of hair follicles was reduced by about 22% compared to the normal group. However, it was confirmed that in all cases of administering mixtures of mixing sulforaphane, indole-3-carbinol and β-sitosterol at various mixing ratios, the density of hair follicles was significantly increased, compared to the negative control group.

**Example 5-5. Confirmation of effect according to administration method of compounds**

To the experimental animals bred in Example 2-1, a mixture of mixing sulforaphane, indole-3-carbinol and β-sitosterol at a ratio of 8:1:1 by the same method as Example 4 was orally administered daily at a dose of 10mg/kg (SFN 8mg/kg + I3C 1mg/kg + S 1mg/kg) in total for 21 days. As a comparative group, the mixture was administered by the same method, and the administration method was a method of intraperitoneal administration (IP). The normal control group (control; normal group) is a group in which neither testosterone nor the extract is administered, and the negative control group (Testosterone; T) is a group in which only testosterone is administered.

Referring to Example 3-2, the density of hair follicles was measured in the skin of the normal control group, negative control group and experimental groups and shown in Table 10 below.

[Table 10]

| Administration method | Density of hair follicles | Rate of change compared to normal group |
|---|---|---|
| Control | 1.25±0.39 | - |
| Testosterone | 0.98±0.23 | -21.6% |
| T+Oral administration | 2.41±0.064 | 92.8% |
| T+IP injection | 1.37±0.051 | 9.6% |

As shown in Table 10 above, in case of the negative control group administered only with the testosterone solution, the density of hair follicles was reduced by about 22% compared to the normal group. It was confirmed that in case of all experimental groups in which the mixture was intraperitoneally administered (IP injection) or orally administered (oral administration), the density of hair follicles was increased, compared to the negative control group, and in particular, in the orally administered experimental group, the density of hair follicles was more significantly increased.

From the above description, those skilled in the art to which the present invention pertains will understand that the present invention may be embodied in other specific forms without changing the technical spirit or essential characteristics thereof. In this regard, it should be understood that the embodiments described above are illustrative in all respects and not restrictive. The scope of the present invention should be construed as all changed or modified forms derived from the meaning and scope of the claims described below and their equivalents rather than the detailed description.

## Claims

**1.** A composition for promoting hair growth or preventing, alleviating or treating hair loss, comprising one or more kinds selected from the group consisting of sulforaphane, indole-3-carbionol, beta-sitosterol (β-sitosterol), and salts thereof

as an active ingredient.

2. A composition for promoting hair growth or preventing, alleviating or treating hair loss, comprising a broccoli (Brassica oleracea var. italica) extract, an avocado (Persea americana) extract or a mixture thereof as an active ingredient.

3. The composition for promoting hair growth according to claim 2, wherein the broccoli extract is extracted with one or more kinds of extraction solvents selected from the group consisting of water and straight chain or branched chain alcohols having 1 to 4 carbon atoms, and
the avocado extract is extracted with a petroleum ether solution.

4. A cosmetic composition for promoting hair growth or preventing, alleviating or treating hair loss, comprising one or more kinds selected from the group consisting of sulforaphane, indole-3-carbionol, beta-sitosterol (β-sitosterol), and salts thereof as an active ingredient.

5. A cosmetic composition for promoting hair growth or preventing, alleviating or treating hair loss, comprising a broccoli (Brassica oleracea var. italica) extract, an avocado (Persea americana) extract or a mixture thereof as an active ingredient.

6. The cosmetic composition for promoting hair growth according to claim 5, wherein the broccoli extract is extracted with one or more kinds of extraction solvents selected from the group consisting of water and straight chain or branched chain alcohols having 1 to 4 carbon atoms, and
the avocado extract is extracted with a petroleum ether solution.

7. A health functional food for promoting hair growth or preventing, alleviating or treating hair loss, comprising one or more kinds selected from the group consisting of sulforaphane, indole-3-carbionol, beta-sitosterol (β-sitosterol), and salts thereof.

8. A health functional food for promoting hair growth or preventing, alleviating or treating hair loss, comprising a broccoli (Brassica oleracea var. italica) extract, an avocado (Persea americana) extract or a mixture thereof.

9. The health functional food according to claim 8, wherein the broccoli extract is extracted with one or more kinds of extraction solvents selected from the group consisting of water and straight chain or branched chain alcohols having 1 to 4 carbon atoms, and
the avocado extract is extracted with a petroleum ether solution.

【FIG. 1a】

2500
2000
1500
1000
500
0
Testosterone (pg/ml)
Group 1
Group 2
Group 3
Group 4
Group 5

【FIG. 1b】

200
150
100
50
0
Dihydrotestosterone (pg/ml)
Group 1
Group 2
Group 3
Group 4
Group 5

【FIG. 2】

Group 1
Group 2
Group 3
Group 4
Group 5

【FIG. 3】

Length of hair follicle (mm)
2
1.8
1.6
1.4
1.2
1
0.8
0.6
0.4
0.2
0
Group 1
Group 2
Group 3
Group 4
Group 5

【FIG. 4a】

Testosterone (pg/ml)
2500
2000
1500
1000
500
0
Control
Testosterone
T+SFN
T+I3C
T+β-sitosterol
T+SFN+I3C+β-sitosterol

【FIG. 4b】

Dihydrotestosterone (pg/ml)
200
180
160
140
120
100
80
60
40
20
0
Control
Testosterone
T+SFN
T+I3C
T+β-sitosterol
T+SFN+I3C+β-sitosterol

【FIG. 5】

Length of hair follicle (mm)
2
1.8
1.6
1.4
1.2
1
0.8
0.6
0.4
0.2
0
Control
Testosterone
T+SFN
T+I3C
T+β-sitosterol
T+SFN+I3C+β-sitosterol

**INTERNATIONAL SEARCH REPORT**

International application No.
**PCT/KR2021/013668**

**A. CLASSIFICATION OF SUBJECT MATTER**

**A23L 33/10**(2016.01)i; **A23L 33/105**(2016.01)i; **A23L 5/20**(2016.01)i; **A23L 29/00**(2016.01)i; **A61K 31/265**(2006.01)i; **A61K 31/404**(2006.01)i; **A61K 31/575**(2006.01)i

According to International Patent Classification (IPC) or to both national classification and IPC

**B. FIELDS SEARCHED**

Minimum documentation searched (classification system followed by classification symbols)

A23L 33/10(2016.01); A61K 31/404(2006.01); A61K 31/575(2006.01); A61K 31/704(2006.01); A61K 36/25(2006.01); A61K 36/73(2006.01); A61K 36/9068(2006.01); A61K 8/97(2006.01); A61P 17/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 설포라판(sulforaphane), 인돌-3-카비놀(indole-3-carbinol), 베타-시토스테롤(β-sitosterol), 브로콜리(Brassica oleracea), 아보카도(Persea americana), 발모(depilation), 탈모(hair loss)

**C. DOCUMENTS CONSIDERED TO BE RELEVANT**

| Category* | Citation of document, with indication, where appropriate, of the relevant passages | Relevant to claim No. |
|---|---|---|
| X<br>A | SASAKI, M. et al. Sulforaphane promotes murine hair growth by accelerating the degradation of dihydrotestosterone. Biochemical and Biophysical Research Communication. 2016, vol. 472, pp. 250-254.<br>See abstract; page 251, column (right); and figures 1-4. | 1,7<br>2-5,6,8,9 |
| X | KR 10-1440061 B1 (JEONNAM BIOINDUSTRY FOUNDATION) 17 September 2014 (2014-09-17)<br>See paragraph [0014]; experimental example; formulation examples 1 and 2; and figure 6. | 1,4 |
| X<br>Y | KR 10-1721685 B1 (ANYDOCTOR HEALTHCARE CO., LTD.) 30 March 2017 (2017-03-30)<br>See paragraphs [0015] and [0022]; and claim 1. | 2,5,8<br>3,6,9 |
| Y | US 2007-0110705 A1 (MARINOS, E.) 17 May 2007 (2007-05-17)<br>See claims 1 and 8. | 3,6,9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 January 2022** | **13 January 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2021/013668**

**C. DOCUMENTS CONSIDERED TO BE RELEVANT**

| Category* | Citation of document, with indication, where appropriate, of the relevant passages | Relevant to claim No. |
|---|---|---|
| Y | CARLOS, C.-V. et al. Quantitative Genetic Analysis of Three Important Nutritive Traits in the Fruit of Avocado. J. Amer. Soc. Hort. Sci. 2013, vol. 138, no. 4, pp. 283-289. See abstract; and page 284. | 3,6,9 |
| X | JP 2011-524412 A (BOURAS, E.) 01 September 2011 (2011-09-01) See paragraphs [0039]-[0041] and [0050]. | 1,4,7 |
| X | KR 10-2011-0001290 A (CHUNGBUK HEALTH SCIENCE UNIVERSITY et al.) 06 January 2011 (2011-01-06) See claim 1. | 1,4,7 |
| A | CN 105853410 A (ZHANG, Y.) 17 August 2016 (2016-08-17) See claim 1. | 1,4,7 |

Form PCT/ISA/210 (second sheet) (July 2019)

INTERNATIONAL SEARCH REPORT

International application No.
**PCT/KR2021/013668**

**Box No. III Observations where unity of invention is lacking (Continuation of item 3 of first sheet)**

This International Searching Authority found multiple inventions in this international application, as follows:

The invention of group 1: claims 1, 4 and 7 pertain to a use invention of sulforaphane, indole-3-carbinol, β-sitosterol and a salt thereof,
The invention of group 2: claims 2, 3, 5, 6, 8 and 9 pertain to a use invention of a broccoli extract, an avocado extract or a mixture thereof.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2019)

**INTERNATIONAL SEARCH REPORT**
**Information on patent family members**

International application No.
**PCT/KR2021/013668**

| Patent document cited in search report | Publication date (day/month/year) | Patent family member(s) | Publication date (day/month/year) |
|---|---|---|---|
| KR 10-1440061 B1 | 17 September 2014 | None | |
| KR 10-1721685 B1 | 30 March 2017 | KR 10-2016-0135532 A | 28 November 2016 |
| US 2007-0110705 A1 | 17 May 2007 | CA 2588874 A1 | 16 June 2005 |
| | | EP 1758542 A1 | 07 March 2007 |
| | | GR 1004732 B | 26 November 2004 |
| | | WO 2005-053625 A1 | 16 June 2005 |
| JP 2011-524412 A | 01 September 2011 | AT 538799 T | 15 January 2012 |
| | | AU 2009-269822 A1 | 14 January 2010 |
| | | BR PI0914947 A2 | 20 October 2015 |
| | | CA 2724318 A1 | 14 January 2010 |
| | | CN 102046183 A | 04 May 2011 |
| | | CN 102046183 B | 13 November 2013 |
| | | EA 017856 B1 | 29 March 2013 |
| | | EA 201071369 A1 | 30 August 2011 |
| | | EP 2296668 A1 | 23 March 2011 |
| | | EP 2296668 B1 | 28 December 2011 |
| | | GB 2461037 A | 23 December 2009 |
| | | KR 10-2011-0028494 A | 18 March 2011 |
| | | US 2011-0076328 A1 | 31 March 2011 |
| | | US 9572826 B2 | 21 February 2017 |
| | | WO 2010-004303 A1 | 14 January 2010 |
| | | ZA 201008160 B | 27 July 2011 |
| KR 10-2011-0001290 A | 06 January 2011 | None | |
| CN 105853410 A | 17 August 2016 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200129117
- KR 1020200129118


### Non-patent literature cited in the description


- *CHEMICAL ABSTRACTS,* 4478-93-7
- *CHEMICAL ABSTRACTS,* 700-06-1
- *CHEMICAL ABSTRACTS,* 83-46-5
- *CHEMICAL ABSTRACTS,* 101316-46-5